# EUROPÄISCHE PATENTSCHRIFT

(11) **EP 0 482 325 B1**
(45) Veröffentlichungstag und Bekanntmachung des Hinweises auf die Patenterteilung: **13.09.1995**
(21) Anmeldenummer: 91114984.7
(22) Anmeldetag: 05.09.1991
(51) Int. Cl.: C07H 15/04

(54) **Verfahren zur Herstellung von Alkylglycosiden und Alkyloligoglycosiden**
Process for manufacturing alkyl glycosides and alkyl oligoglycosides
Procédé de production de glycosides d'alkyle et d'oligoglycosides d'alkyle

(30) Priorität: 26.10.1990 DE 4034074
(43) Veröffentlichungstag der Anmeldung: 29.04.1992
(73) Patentinhaber: HÜLS AKTIENGESELLSCHAFT, 45764 Marl (DE)
(72) Erfinder: Oberholz, Alfred, Dr., W-4370 Marl (DE); Kahsnitz, John, Dr., W-4358 Haltern (DE); Schmidt, Stefan, Dr., W-4350 Recklinghausen (DE)

(56) Entgegenhaltungen:
- EP-A- 0 099 183
- EP-A- 0 231 890
- DE-A- 3 001 064
- DE-A- 3 623 246

## Beschreibung

Die Erfindung betrifft ein Verfahren zur Herstellung von Alkylglycosiden und Alkyloligoglycosiden, bei dem man Saccharide in wäßriger Lösung mit Alkoholen mit 1 bis 6 Kohlenstoffatomen unter Säurekatalyse bei erhöhter Temperatur umsetzt.

Alkylglycoside und Alkyloligoglycoside mit Alkylresten mit 1 bis 6 Kohlenstoffatomen sind Zwischenprodukte bei der Herstellung von langkettigen Alkylpolyglycosiden, die Tensideigenschaften besitzen.

Die Herstellung von Alkylglucosiden aus wäßrigen Sacchariden und kurzkettigen Alkoholen erfolgt nach EP-A-0 099 183 bei erhöhtem Druck und vorzugsweise in Gegenwart von Cosolvenzien. Die Reaktion kann in einem Rührkessel oder in einem Rohrreaktor erfolgen, wobei die Umsetzung vorzugsweise in einem Rührkessel vorgenommen wird.

In DE-A-36 23 246 wird bei der Reaktion von wäßrigem Saccharid und kurzkettigem Alkohol kein Cosolvens benötigt. Die Reaktion kann in kontinuierlichen oder diskontinuierlichen Rührkesseln durchgeführt werden. Vorzugsweise wird eine Rührkesselkaskade verwendet. Dieses Verfahren liefert Produkte, die zum Teil hohen Anforderungen hinsichtlich der Farbe noch nicht genügen.

Aufgabe der vorliegenden Erfindung ist es, ohne Anwendung von Überdruck und fremden Cosolvenzien farblich gute Alkylglycoside und Alkyloligoglycoside aus Sacchariden in wäßriger Lösung und Alkoholen mit 1 bis 6 Kohlenstoffatomen herzustellen.

Die Aufgabe wird erfindungsgemäß dadurch gelöst, daß man die Reaktion in einer Gegenstromreaktionskolonne durchführt.

Als Saccharide sind Mono-, Di- oder Oligosaccharide geeignet. Beispiele dafür sind Glucose, Mannose, Galactose, Gulose, Allose, Ribose, Arabinose, Xylose oder Saccharose. Es können auch Stärkehydrolysate eingesetzt werden. Allgemein werden pumpfähige wäßrige Lösungen eingesetzt. Saccharidsirupe mit 10 bis 75 % Wasser werden dabei vorzugsweise verwendet. Dextrosesirupe mit Wassergehalten von 50 bis 70 % werden ganz besonders bevorzugt.

Geeignete Alkohole sind beispielsweise Methanol, Ethanol, Propanol, Isopropanol, n-Butanol, Isobutanol, Pentanol, Hexanol oder Cyclohexanol. Vorzugsweise wird n-Butanol eingesetzt.

Als Katalysatoren kommen vor allem Mineralsäuren wie Schwefel- oder Phosphorsäure sowie starke organische Säuren wie Benzolsulfonsäure, Cumolsulfonsäure oder p-Toluolsulfonsäure in Betracht. Wegen einer geringeren korrosiven Wirkung werden als Katalysatoren vorzugsweise organische Säuren eingesetzt. Die Katalysatoren werden meist als wäßrige oder als alkoholische Lösung zugegeben.

Die Alkyloligoglycoside haben im allgemeinen einen Oligomerisationsgrad im Bereich von 1 bis 4.

Als Gegenstromreaktionskolonne können übliche Kolonnen, wie beispielsweise Glockenböden-, Siebböden-, Tunnelböden- oder Füllkörperkolonnen verwendet werden. Die Kolonnen besitzen vorzugsweise 3 bis 40 praktische Böden. Dabei werden Kolonnen mit 10 bis 20 praktischen Böden ganz besonders bevorzugt.

In der Gegenstromreaktionskolonne wird eine Temperatur eingestellt, bei der Saccharid, Alkylglycosid und Alkyloligoglycosid in der Flüssigphase ablaufen können und bei der Alkohol und Wasser als Gasphase am Kopf der Kolonne abgezogen werden können. Die Reaktionstemperatur beträgt meist 70 bis 150 °C. Dabei werden Temperaturen von 90 bis 140 °C vorzugsweise eingestellt.

Obwohl die Reaktion auch bei Überdruck durchgeführt werden kann, erfolgt die Reaktion im allgemeinen bei Normaldruck oder bei leichtem Vakuum bis etwa 0,05 MPa.

Das Verfahren erfordert keine fremden Cosolvenzien und keinen Überdruck. Der apparative Aufwand ist gering. Die Apparatur ist einfacher als eine Rührkesselkaskade. Man benötigt keine mechanische Energien. Außerdem liefert die Kolonne gegenüber der Kaskade bei hohem Umsatz farblich verbesserte Produkte.

Erfindungsgemäß wird im allgemeinen so verfahren, daß man die wäßrige Saccharidlösung, die auch alkoholisches Alkylglycosid enthalten kann, sowie die Katalysatorlösung in den oberen Teil der Gegenstromreaktionskolonne einführt. Der Alkohol wird dampfförmig in den unteren Teil der Kolonne eingeleitet. Nach der Reaktion wird unumgesetzter Alkohol und Wasser am Kopf der Kolonne abgezogen. Erfolgt beim Abkühlen des Kopfproduktes Phasentrennung, so kann der Alkohol direkt oder nach Aufarbeitung zurückgeführt werden. Der Alkohol kann auch zusammen mit dem frischen Alkohol dampfförmig in den unteren Teil der Kolonne eingeleitet werden. Das Reaktionsprodukt, Alkylglycoside und Alkyloligoglycoside, wird am Sumpf der Kolonne isoliert.

Wenn Alkylglycosid in den oberen Teil der Kolonne eingeführt werden soll, so kann man dazu einen Teil des Reaktionsproduktes zurückführen. Man kann das Alkylglycosid beispielsweise aber auch in einer Vorreaktor-Stufe (Rührkessel oder Rührkesselkaskade) aus Saccharid und Alkohol herstellen. Für diese Vorreaktion kann der Alkohol, den man am Kopf der Kolonne nach der Wasserabscheidung erhält, eingesetzt werden.

In den folgenden Beispielen sind Prozentangaben, wenn nicht anders angegeben, Angaben in Gewichtsprozent.

### Beispiel 1

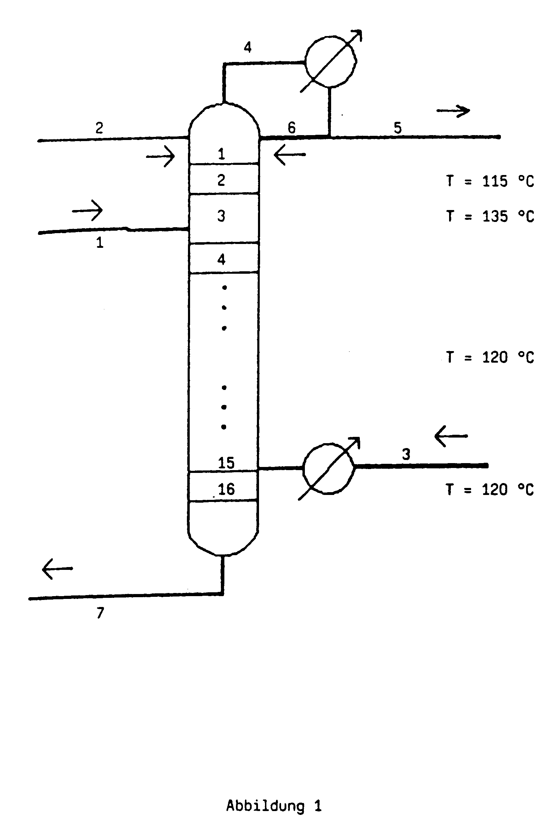

Die Reaktion wird in einer in Abbildung 1 schematisch dargestellten Glockenbödenkolonne (16 praktische Böden, statistisches Hold-up = 200 ml/Boden, Innendurchmesser der Böden D = 80 mm, Höhe h = 100 mm/Boden) durchgeführt. Über Leitung 1 werden 2,3 mol/h Glucose als 55%ige wäßrige Lösung und 8 l/h 35%iges butanolisches Butylglucosid (10,9 mol/h) auf den 3. Boden gegeben. 108 ml/h 10%ige butanolische p-Toluolsulfonsäure werden über Leitung 2 dem Kopf der Kolonne zugeführt. Außerdem werden 1,3 l/h Butanol (14,4 mol/h) als überhitzter Dampf auf den 15.

Boden in die Kolonne geleitet. Die Kolonne wird bei den in Abbildung 1 angegebenen Temperaturen betrieben.

Ein Butanol/Wasser-Gemisch wird über Leitung 4 als Destillat abgezogen. Nach Abkühlung und Phasentrennung wird Wasser über Leitung 5 abgetrennt, während Butanol über Leitung 6 auf die Kolonne zurückgegeben wird. Aus der kalten Blase der Kolonne werden 10 l/h butanolisches Butylglucosid mit einem Glucosegehalt von < 0,5 % und einem Wassergehalt von < 0,6 % über Leitung 7 gewonnen.
Jodfarbzahl: < 15

### Vergleichsbeispiel A

### Herstellung von Butylglucosid in einer Rührkesselkaskade

In einer 2stufigen Rührkesselkaskade, die aus zwei 5-l-Rührreaktoren besteht, werden 0,8 l/h 70%iger Glucosesirup (3,45 mol/h), 1,5 l/h Butanol (16,8 mol/h) sowie stündlich 1 Gewichtsprozent p-Toluolsulfonsäure, bezogen auf die Gesamtmenge an Einsatzstoffen, eingeleitet. Die Rührreaktoren werden auf Siedetemperatur erhitzt, so daß kontinuierlich ein Butanol/Wasser-Gemisch abdestilliert. Das Destillat wird in eine Wasserphase und eine Butanolphase aufgetrennt, wobei die Butanolphase in die Reaktoren zurückgeleitet und die Wasserphase verworfen wird. Das Produkt aus dem zweiten Reaktor ist eine etwa 35%ige Lösung von Butylglucosid in Butanol mit einem Glucosegehalt von < 0,5 % und einem Wassergehalt von < 0,6 %.
Jodfarbzahl: > 300

## Patentansprüche

1. Verfahren zur Herstellung von Alkylglycosiden und Alkyloligoglycosiden durch säurekatalysierte Umsetzung von Sacchariden in wäßriger Lösung mit Alkoholen mit 1 bis 6 Kohlenstoffatomen bei 70-150°C,
dadurch gekennzeichnet,
daß man die Reaktion in einer Gegenstromreaktionskolonne durchführt.

2. Verfahren nach Anspruch 1,
dadurch gekennzeichnet,
daß man einen Saccharidsirup mit 10 bis 75 % Wasser einsetzt.

3. Verfahren nach Anspruch 2,
dadurch gekennzeichnet,
daß man einen Dextrosesirup mit 50 bis 70 % Wasser verwendet.

4. Verfahren nach Anspruch 1,
dadurch gekennzeichnet,
daß man als Alkohol n-Butanol verwendet.

5. Verfahren nach Anspruch 1,
dadurch gekennzeichnet,
daß man die Reaktion in einer Kolonne mit 3 bis 40 praktischen Böden durchführt.

## Claims

1. A process for the preparation of alkyl glycosides and alkyl oligoglycosides by acid-catalysed reaction of saccharides in aqueous solution with alcohols having 1 to 6 carbon atoms at 70-150°C, characterised in that the reaction is carried out in a counter-current reaction column.

2. A process according to claim 1, characterised in that a saccharide syrup containing 10 to 75% of water is used.

3. A process according to claim 2, characterised in that a dextrose syrup containing 50 to 70% of water is used.

4. A process according to claim 1, characterised in that the alcohol used is n-butanol.

5. A process according to claim 1, characterised in that the reaction is carried out in a column having 3 to 40 actual plates.

## Revendications

1. Procédé pour préparer des alkylglycosides et des alkyloligoglycosides par réaction, catalysée par un acide, de saccharides en solution aqueuse avec des alcools ayant de 1 à 6 atomes de carbone à une température de 70 à 150°C,
caractérisé en ce que la réaction est mise en oeuvre dans une colonne de réaction à contre-courant.

2. Procédé selon la revendication 1,
caractérisé en ce qu'on utilise un sirop de saccharide ayant de 10 à 75 % d'eau.

3. Procédé selon la revendication 2,
caractérisé en ce qu'on utilise un sirop de dextrose contenant de 50 à 70 % d'eau.

4. Procédé selon la revendication 1,
caractérisé en ce qu'on utilise comme alcool le n-butanol.

5. Procédé selon la revendication 1,
caractérisé en ce qu'on met en oeuvre la réaction dans une colonne ayant de 3 à 40 plateaux pratiques.
